# EUROPEAN PATENT SPECIFICATION

(11) **EP 1 706 070 B1**
(45) Date of publication and mention of the grant of the patent: **23.02.2011**
(21) Application number: 05711870.5
(22) Date of filing: 18.01.2005
(51) Int. Cl.: A61F 2/06

(54) **MULTIPLE STITCHES FOR ATTACHING STENT TO GRAFT**
MEHRERE STICHE ZUM BEFESTIGEN EINES STENTS AN EINER PROTHESE
DE MULTIPLES SUTURES PERMETTANT DE FIXER UN STENT A UNE GREFFE

(30) Priority: 20.01.2004 US 537565 P
(43) Date of publication of application: 04.10.2006
(73) Proprietor: Cook Incorporated, Bloomington, Indiana 47404 (US)
(72) Inventor: OSBORNE, Thomas, A., Bloomington, IN 47401 (US); DEBRUYNE, Michael, P., Bloomington, IN 47408 (US); LAD, Ashvin, Indianapolis, IN 46220 (US)
(74) Representative: Powell, Stephen David
(86) International application number: PCT/US2005/002108
(87) International publication number: WO 2005/070338

(56) References cited:
- EP-A- 1 029 517
- WO-A-2004/002365
- US-A- 5 824 044
- US-A- 6 015 431
- US-A- 6 077 297

## Description

### FIELD OF THE INVENTION

The field of the invention is that of stents and grafts and the attachment of stents to grafts useful for insertion into a body passageway, such as a vascular passageway or other body lumen.

### BACKGROUND

Stent-grafts are used in body lumens and in particular, in vascular lumens. These grafts allow the use of the lumen in situations where the lumen may have a defect, such as a stenosis or an aneurysm. Stents perform functions that make them useful in combination with grafts. An external stent can resist pressure on a graft, and help to provide support and containment for a blood vessel. An internal stent can provide an outward pressure to keep the graft and hence the lumen open. The stent also may be used to anchor the graft in place, provided there are anchors or barbs on the, stent that secure the stent, and hence the graft, in the desired location.

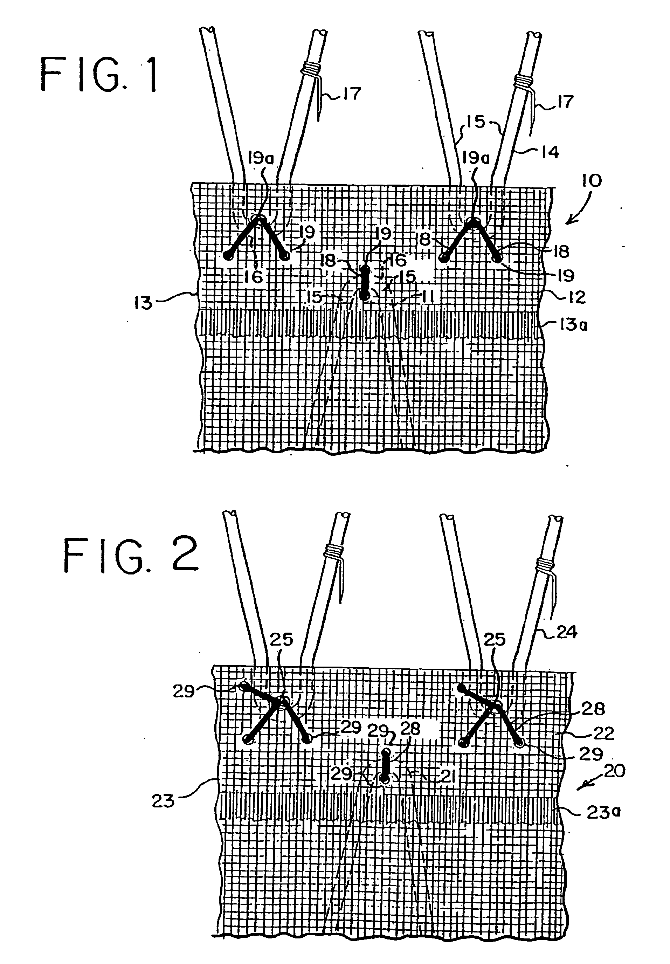

Previous grafts have included stents, but sometimes have defects that hinder their use. For example, Fig. 2 of U. S. Pat. No. 6,004, 347 depicts a stent that may be difficult to manufacture because of the many resistance welds. A more serious difficulty is the method used to attach the stent to the graft, as depicted in the patent. In order to properly secure the stent to the graft, according to the patent, eight penetrations or openings are needed for four successive stitches. Each stitch is positioned further away from a bend in the proximal end of the stent. This method spreads out the stress on the graft from the stent, but requires many penetrations per bend of the stent.

Figs. 17-18 of U. S. Pat. No. 6,287, 330 and Figs. 19-21 of U. S. Pat. No. 6,221, 102 depict stents used to secure a graft material. In these examples, the stent is separate from an attachment system that anchors the graft to the body lumen. Many stitches are required to secure the stent and the attachment system to the graft.

EP-A 1029517 discloses an endoluminal stent graft in which a frame is secured to a liner by sutures.

US 2003/0176912 discloses a stent graft having holes through the graft material for the attachment of eyelets. The disclosure of this document corresponds to the preambles of claims 1 and 12.

### BRIEF SUMMARY

One aspect of the invention is an endoluminal prosthetic device, comprising: a graft comprising warp and weft threads, and a stent comprising a plurality of struts and apices between the struts, characterised in that at least one of the apices is secured to the graft by at least two stitches between the threads of the graft and within five graft threads of each other.

In one embodiment the stent is an anchoring stent and the device further comprises a second stent secured with stitches to the graft.

Another aspect of the invention is a method of forming an endoluminal prosthetic device, the method comprising: providing a graft having warp and weft threads; and joining a first stent to the graft, the first stent comprising a plurality of struts and apices between the struts, characterised in that the first stent is joined to the graft by at least two stitches between the threads of the graft and within five graft threads of each other.

There are many ways to practice the present invention, as shown in the following drawings and specification. The embodiments are not meant to limit the invention, but rather to describe and illustrate some of the ways that the present invention may be used.

### BRIEF DESCRIPTION OF THE DRAWING

Fig. 1 is an elevational view of a first embodiment;

Fig. 2 is an elevational view of a second embodiment;

Fig. 3 is an elevational view of a third embodiment;

Fig. 4 is an elevational view of a further embodiment; and

Fig. 5 depicts an apparatus to deploy a prosthesis or prosthetic device.

### DETAILED DESCRIPTION OF THE PREFERRED EMBODIMENTS

An endoluminal prosthetic device in accordance with the present disclosure is presented in Fig. 1. The device 10 comprises a graft 12 made from polyester or other graft material suitable for use in a human body. These materials may include, but are not limited to, polyester, polyurethane, polyethylene, polypropylene, and polytetrafluoroethylene, as well as other fluorinated polymer products. A preferred material is polyester, woven in a twill pattern, and available commercially from Vascutek Ltd. of Scotland. Biomaterials may also be used, such as collagen or ECM (extracellular matrix materials). One example is porcine small intestine submucosa (SIS) material, which may be remodeled into repair tissues for the human body.

Device 10 also includes at least one anchoring stent 14 and an internal stent 11. These stents may be in the form of zigzag stents, that is, a continuous chain of struts 15 and joints between the struts, or apices 16. Zigzag stents sold under the trademark Cook-Z^{®} and stent-grafts for abdominal aortic aneurysm (AAA) repair, made by Cook Incorporated of Bloomington, Indiana, are an example of commercial embodiments. Serpentine stents, having curved struts, may also be used.

In addition, anchoring stent 14 may include anchors 17, small barbs or hooks that will anchor the stent to the internal wall of a body lumen, such as a wall of an aorta. Anchoring stents are oriented so that they may easily attach to the body lumen when they are placed inside a patient. The barbs are preferably attached to strut 15 by welding, soldering, or other permanent attachment. The barb is preferably attached by several coils at a first pitch or spacing, and a final coil at a greater pitch than the first coils, to increase the fatigue life of the barb.

The stents may be made from one or more of several materials. These materials include, but are not limited to, stainless steel, titanium, and shape memory materials, such as Nitinol. These stents are desirably in the form of a zigzag stent, a continuous chain of struts and intersections where the struts join. As noted above, anchoring stent 14 may have one or more anchors 17, or small barbs, for securing the anchoring stent in a body lumen or passageway. Radially compressible, self expanding stents are preferred. PCT Application WO 98/53761 discloses a number of details concerning stents, stent grafts, and a method for implanting stent grafts into the human body.

The graft, as discussed above, should be suitable for placement inside a person, although these grafts are not limited to uses for humans and may also be used for animals. The graft 12, as depicted in Fig. 1, desirably includes a short cuff 13. This cuff doubles the thickness of the graft and thus reinforces the graft in what may be the area of greatest stress. The end of the cuff forms a continuous band of fuzzy, frayed material 13a. The fraying encourages ingrowth of tissue to the graft. A cuff of from about 1,27 mm (0.050 inches) to about 1,27 cm (0.50 inches) long may be used.

Anchoring stent 14 is secured to graft 12 using sutures 18 through penetrations 19 in the graft. Anchoring stent 14 is desirably secured to graft 12 with more than one stitch or suture 18 in order to strengthen its attachment to the graft. In order to minimize the number of penetrations in the graft, it is desirable for the sutures to share penetrations or openings in the graft to the greatest extent possible. Thus, in Fig. 1, anchoring stent 14 is secured with two stitches and three penetrations, shown at 19, 19a, for each of two struts 15 and single joint or apex 16 of anchoring stent 14. The stitches, therefore, share openings or penetrations where possible, as shown at penetration 19a. Note that the openings or penetrations are shown greatly exaggerated for clarity in all of the drawings.

In the example of Fig. 1, each segment of the anchoring stent includes two struts 15 and apex 16. Each segment is secured with two stitches using three penetrations. Of course, it is possible to have fewer penetrations, such as two, but then there may only be one stitch. However, in this embodiment, it is desired to secure the anchoring stent with more than one stitch per apex or segment. Note that in Fig. 1, each segment of internal stent 11 may be secured to graft 12 with only one stitch 18 using two penetrations or holes 19 in graft 12. The stresses on the internal or other external stents are typically not as great as those involved with the anchoring stent. Each suture or stitch preferably includes at least one knot, so that once the suture is made, the suture will be secure in its position and will not move.

In practicing the invention, it is preferable to push the needle holding the suture through precisely the same penetration in order to minimize the size of the opening or penetration. Preferably, a seamstress or technician assembling the stent graft of the present invention will place the needle and the resulting suture through precisely the same point between the threads of the graft, so that two or more sutures share precisely the same opening or space through the warp and weft threads of the graft. Sharing a penetration may also mean, however, that the subsequent penetration and stitch is within a few graft threads, as shown in penetration 19a. Therefore, when two stitches share a penetration in the graft, it means the stitches are in precisely the same opening or are sufficiently close so that the stitches are separated by 0 to about 5 of the graft threads.

The embodiment described in Fig. 1 provides excellent retention of stents within the graft or to the graft, while minimizing the number of openings or penetrations needed for sutures. Thus, the potential for leakage of body fluid, such as blood, is minimized. In addition, the potential for thrombi and other disruptions is minimized by minimizing the number of growth sites.

The suture may be made of any biocompatible fiber suitable for the application, including but not limited to, monofilament polyester or braided multi- filament polyester, nylon, polyaramid, polypropylene, and polyethylene. Green, braided polyester 4-0 suture material is preferred for attaching internal stents to grafts, while monofilament suture material is preferred for attaching anchoring stents to grafts. The polyester 4-0 suture material is nonabsorbable and has limits of 0.150 to 0.199 mm (metric size 1.5). Suture materials are commercially available from a number of companies, including Genzyme Biosurgery, Fall River, MA. The suture material may be attached to a hollow needle used to thread the suture through the graft, thus attaching the stent to the graft. In making the sutures at the apices described above, suturing may begin at any convenient point.

In the present context, suture means the thread or material that secures the stents to the graft. Suture may also refer to the configuration of this material in a loop, the material securing a portion of a stent to a graft. A suture is made by looping material through the graft and around the stent or an apex of a stent. A knot is then tied, intertwining the ends of the suture in such a way that they will not be easily separated. A suture thus has a knot and may have more than one knot. In some cases, or in many cases, an apex of a stent may be secured to the graft by two loops of suture or thread through the graft and also through or around the apex of the stent, and then secured with one knot or more than one knot, in essence tying the ends of the suture. Knots may be locking knots, preferred for apices, or overthreaded knots, preferred along the length of the strut or for other applications. Knots in the stent-gran may include any other useful or desired knots and are not limited to these types. A stitch is a single suture and includes at least one knot.

Fig. 2 is a second embodiment of an endoluminal prosthetic device 20, including graft 22, cuff 23 and frayed edge 23a. Internal stent 21 and anchoring stent 24 are secured to graft 22. The segment of internal stent 21 is secured with a single suture or stitch 28, using two openings or penetrations 29. Each segment of anchoring stent 24 is secured to graft 22 with three stitches 28, using four penetrations or openings 25, 29. In this embodiment, anchoring stent 24 is better secured against stresses that would tend to separate anchoring stent 24 from graft 22. In the event of a suture or stitch failure, all three stitches 28 at any one apex or segment of the anchoring stent would have to fail in order for that portion of the stent to separate from the graft. Thus, the stent is better secured to the graft than if only one stitch were used. Because the stitches or sutures share openings or penetrations 25, there are fewer openings or penetrations in the graft, and leakage and reactive sites within the graft are minimized. All openings or penetrations are shown in greatly exaggerated size, as compared with the stitches and other components of the endoluminal prosthetic device.

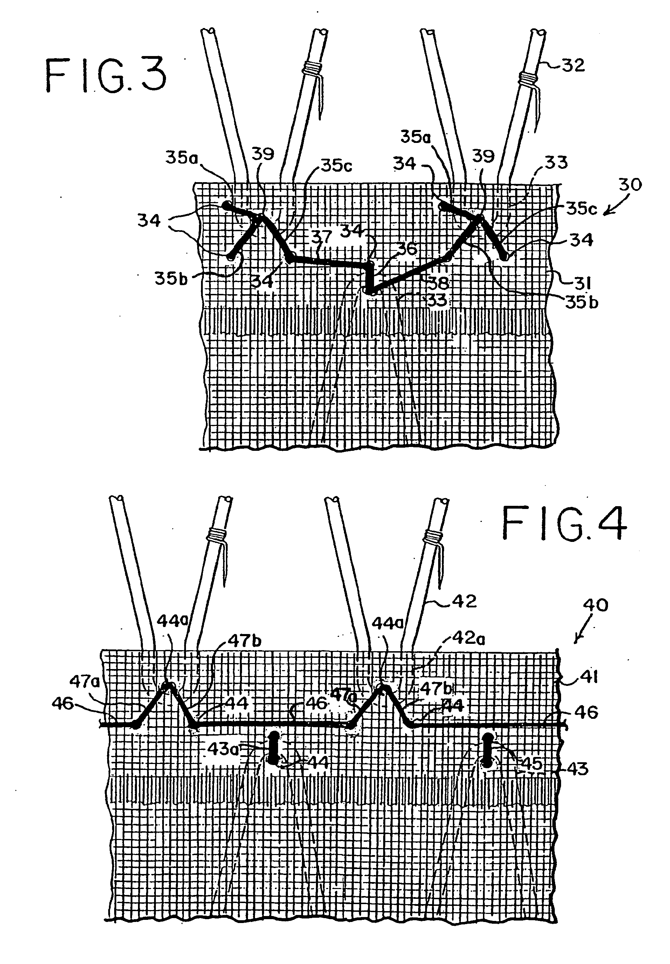

There are other techniques to further increase the strength of the attachment of the stents to the graft. Fig. 3 depicts an endoluminal prosthetic device 30, comprising an aortic graft 31 with an anchoring stent 32 and an internal stent 33. Segments of the anchoring stent 32 are joined to graft 31 with three stitches 35a, 35b, 35c using four openings or penetrations 34, 39. Segments of internal stent 33 are secured to the graft with a single stitch 36 and two penetrations 34 for each apex of the internal stent.

In this embodiment, stitches 35b, 35c, 36 are then joined into a continuous chain as shown with running sutures 37, 38. Running sutures are formed by forming a first stitch or stitches at one point in the stent-graft, tying a knot to secure the stitch or stitches, and then refraining from cutting or trimming the thread or suture material, but "running" the thread or suture material to the next point for a stitch in the stent graft, and then making a stitch or stitches in the next location, and so on, for as many locations as are desired. By using running sutures 37, 38, the anchoring stent is better secured to the graft through its attachment to other stitches. When a stress is placed on anchoring stent 32, the load is distributed through stitches 35 from upper penetrations 39 to lower penetrations 34, transferring the stress from the sutures to graft 31. Running sutures 37, 38, further spread the load to the stitches of adjoining apices. Thus, if a stress is placed on only one or two apices of the anchoring stent, the running sutures help to redistribute the stress to a wider area of the graft. This strengthens the attachment of the stent to the graft.

In practice, the stents may be secured by beginning the stitches with the internal stent, and then running a chain of stitches to the nearest apex or portion of the anchoring stent, and then continuing around the periphery of the graft. It is easier to stitch on the outer periphery of the graft. It may not be possible to have a complete unbroken link of suture around the periphery of the graft, but the chain or running suture should be as complete and continuous as possible.

Fig. 4 is another embodiment using a running suture or continuous chain of sutures around the periphery of the graft. Endoluminal prosthetic device 40 includes a graft 41, an anchoring stent 42 and an internal stent 43. Apices 43a of internal stent 43 are secured to the graft with single stitches 45 through two penetrations 44 per stitch. Apices 42a of anchoring stent 42 are secured to graft 41 by two stitches 47a, 47b using three openings or penetrations 44, 44a. Penetration 44a is formed when the two stitches are made, and the stitches are desirably placed into exactly the same opening or penetration 44a. The sizes of the openings or penetrations are greatly exaggerated in Fig. 4.

The stitches for the anchoring stent are joined by running sutures 46 to link top stitches 47a, 47b. Thus, a running suture 46 is formed by a continuous chain of stitches for anchoring stent 42. This chain strengthens the attachment of the stents to the graft and makes for a stronger attachment of the stent to the graft. When a stress is placed on anchoring stent 42, the load is distributed through both stitches 47a, 47b from penetration 44a to lower penetrations 44, which anchor each apex of the anchoring stent. The stress is thus distributed from the sutures to graft 41. Running sutures 46 further spread the load to the stitches of adjoining apices in the upper stents.

The running suture in Fig. 4 may be formed by first looping thread or suture material around first apex 42a for at least one loop and then tying the thread to form at least one stitch 47a between openings 44 and 44a. Without cutting the thread, and using the same opening or penetration 44a, a second stitch 47b is then formed between opening 44a and the next lower opening 44 by looping the thread around and tying a knot. Without cutting the thread or suture material, the material is then "run" to the next apex 42a of anchoring stent 42 and the process is repeated. The running suture may also include stitches from the internal stent, as shown in Fig. 3, which is achieved by a similar process of stitching. Note that it may not be possible to join all the stitches into a single running suture, as also shown in Fig. 3, in which stitches 35a are not joined in the running suture.

As mentioned above, the top edge of graft 11 may be formed into a cuff, of double thickness, in the top half-inch or top quarter-inch, to add strength to the graft where it is most needed. The sutures are preferably made on an outside periphery of the graft. Sutures or stitches may also be made on an inside periphery of the graft, but the outside is preferred because it is much easier to work on the outside and to leave the "running" portion of the running sutures on the outside of the graft. In addition, it is preferably to leave the inside surfaces of the graft as free from interruptions as possible, in order to avoid thrombi and to encourage flow of body fluids to the greatest extent possible.

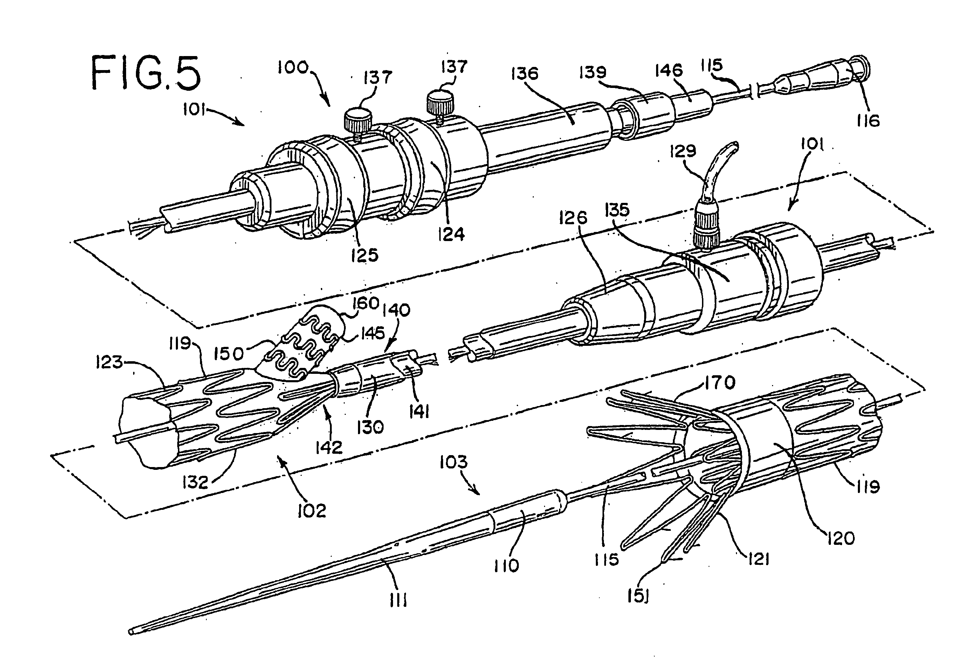

Figure 5 shows a self-expanding bifurcated prosthesis 120, a self-expanding tubular prosthesis 150, and an endovascular deployment system 100, also known as an introducer 100, for deploying the prosthesis 120 in a lumen of a patient during a medical procedure. These items are each described in greater detail in PCT application WO98/53761. The terms "proximal" and "proximally" are used for a position or direction towards the patient's heart and the terms "distal" and "distally" are used for a position or direction away from the patient's heart.

The bifurcated prosthesis 120 has a generally inverted Y-shaped configuration. The prosthesis 120 includes a body 123, a shorter leg 160 and a longer leg 132. The bifurcated prosthesis 120 comprises a tubular graft material, such as polyester, with self-expanding stents 119 attached thereto. The self-expanding stents 119 cause the prosthesis 120 to expand following its release from the introducer 100. The prosthesis 120 also includes a self-expanding zigzag stent 121 that extends from its proximal end. The self-expanding zigzag stent 121 has distally extending barbs 151. When it is released from the introducer 100, the self expanding zigzag stent 121 anchors the barbs 151, and thus the proximal end of the prosthesis 120, to the lumen of the patient.

The self-expanding tubular prosthesis 150 is similar to the bifurcated prosthesis 120, but has a unitary (*i.e*. non-bifurcated) lumen. The tubular prosthesis 150 also comprises a tubular graft material, such as polyester, having self-expanding stents attached thereto. The tubular prosthesis 150 is configured to connect to the shorter leg 160 of the bifurcated prosthesis 120.

The introducer 100 includes an external manipulation section 101, a distal attachment region 122 and a proximal attachment region 103. The distal attachment region 102 and the proximal attachment region 103 secure the distal and proximal ends of the prosthesis 120, respectively. During the medical procedure to deploy the prosthesis 120, the distal and proximal attachment regions 102 and 103 will travel through the lumen to a desired deployment site. The external manipulation section 101, which is acted upon by a user to manipulate the introducer, remains outside of the patient throughout the procedure.

The proximal attachment region 103 of the introducer 100 includes a cylindrical sleeve 110. The cylindrical sleeve 110 has a long tapered flexible extension 111 extending from its proximal end. The flexible extension 111 has an internal longitudinal aperture (not shown). This longitudinal aperture facilitates advancement of the tapered flexible extension 111 along an insertion wire (not shown). The longitudinal aperture also provides a channel for the introduction of medical reagents. For example, it may be desirable to supply a contrast agent to allow angiography to be performed during placement and deployment phases of the medical procedure.

A thin walled metal tube 115 is fastened to the extension 111. The thin walled metal tube 115 is flexible so that the introducer 100 can be advanced along a relatively tortuous vessel, such as a femoral artery, and so that the distal attachment region 102 can be longitudinally and rotationally manipulated. The thin walled metal tube 115 extends through the introducer 100 to the manipulation section 101, terminating at a connection means 116.

The connection means 116 is adapted to accept a syringe to facilitate the introduction of reagents into the thin walled metal tube 115. The thin walled metal tube 115 is in fluid communication with the apertures 112 of the flexible extension 111. Therefore, reagents introduced into connection means 116 will flow to and emanate from the apertures 112.

A plastic tube 141 is coaxial with and radially outside of the thin walled metal tube 115. The plastic tube 141 is "thick walled"- its wall is preferably several times thicker than that of the thin walled metal tube 115. A sheath 130 is coaxial with and radially outside of the plastic tube 141. The thick walled plastic tube 141 and the sheath 130 extend distally to the manipulation region 101.

During the placement phase of the medical procedure, the prosthesis 120 is retained in a compressed condition by the sheath 130. The sheath 130 extends distally to a gripping and haemostatic sealing means 135 of the external manipulation section 101. During assembly of the introducer 100, the sheath 130 is advanced over the cylindrical sleeve 110 of the proximal attachment region 103 while the prosthesis 120 is held in a compressed state by an external force. A distal attachment (retention) section 140 (inside sheath 130 and not visible in this view) is coupled to the thick walled plastic tube 141. The distal attachment section 140 retains a distal end 142 of the prosthesis 120 during the procedure. Likewise, the cylindrical sleeve 110 retains the self-expanding zigzag stent 121.

The distal end 142 of the prosthesis 120 is retained by the distal attachment section 140. The distal end 142 of the prosthesis 120 has a loop (not shown) through which a distal trigger wire (not shown) extends. The distal trigger wire extends through an aperture (not shown) in the distal attachment section 140 into an annular region between the thin walled tube 115 and the thick walled tube 141. The distal trigger wire extends through the annular space to the manipulation region 101. The distal trigger wire exits the annular space at a distal wire release mechanism 125.

The external manipulation section 101 includes a haemostatic sealing means 135. The haemostatic sealing means 135 includes a haemostatic seal (not shown) and a side tube 129. The haemostatic sealing means 135 also includes a clamping collar (not shown) that clamps the sheath 130 to the haemostatic seal, and a silicone seal ring (not shown) that forms a haemostatic seal around the thick walled plastic tube 141. The side tube 129 facilitates the introduction of medical reagents between the thick walled tube 141 and the sheath 130.

A proximal portion of the external manipulation section 101 includes a release wire actuation section that has a body 136. The body 136 is mounted onto the thick walled plastic tube 141. The thin walled tube 115 passes through the body 136. The distal wire release mechanism 125 and the proximal wire release mechanism 124 are mounted for slidable movement onto the body 136.

The positioning of the proximal and distal wire release mechanisms 124 and 125 is such that the proximal wire release mechanism 124 must be moved before the distal wire release mechanism 125 can be moved. Therefore, the distal end 142 of the prosthesis 120 cannot be released until the self-expanding zigzag stent 121 has been released, and the barbs 151 have been anchored to the lumen. Clamping screws 137 prevent inadvertent early release of the prosthesis 120. A haemostatic seal (not shown) is included so that the release wires can extend out through the body 136 without unnecessary blood loss during the medical procedure.

A distal portion of the external manipulation section 101 includes a pin vise 139. The pin vise 139 is mounted onto the distal end of the body 136. The pin vise 139 has a screw cap 146. When screwed in, vise jaws (not shown) of the pin vise 139 clamp against or engage the thin walled metal tube 115. When the vise jaws are engaged, the thin walled tube 115 can only move with the body 136, and hence the thin walled tube 115 can only move with the thick walled tube 141. With the screw cap 146 tightened, the entire assembly can be moved together as one piece.

A second introducer may be used to introduce the tubular prosthesis 150. This second introducer may be based on the same principles as the introducer 100 described above, but less complex. For example, the second introducer may include a complex sheath for containing the tubular prosthesis 150 in a compressed state, so that it can be introduced into a targeted anatomy and then released to either self-expand or be actively expanded with a balloon.

The second introducer may also be adapted so that it can introduce the tubular prosthesis 150 by passing it through one ostium in the bifurcated prosthesis 120 and partially out of another ostium until the terminus of the tubular prosthesis 150 that is closest to the external end of the second introducer is properly positioned. At that point, the tubular prosthesis 150 can be released from the second introducer.

### DEPLOYMENT

Prosthetic modules are preferably deployed seriatim. The intermodular connection between the tubular prosthesis 150 and the bifurcated prosthesis 120 is formed *in situ.* First the bifurcated prosthesis 120 is deployed, and then the tubular prosthesis 150 is deployed. For example, a bifurcated aortic prosthesis 120, as described in WO98/53761, can be deployed into the abdominal aorta. The bifurcated prosthesis 120 has a generally inverted Y-shaped configuration having a body portion 123, a shorter leg 160 and a longer leg 132. The body of the prosthesis is constructed from a tubular woven polyester. At the proximal end of the prosthesis 120 is a self-expanding stent 121 which extends beyond the end of the prosthesis and has distally extending barbs 151. The shorter leg 160 and the longer leg 132 have internal projections extending from their distal termini.

This bifurcated prosthesis 120 can be deployed in any method known in the art, preferably the method described in WO98/53761 in which the device is inserted by an introducer via a surgical cut-down into a femoral artery, and then advanced into the desired position over a stiff wire guide using endoluminal interventional techniques. For example, a guide wire (not shown) is first introduced into a femoral artery of the patient and advanced until its tip is beyond the desired deployment region of the prosthesis 120. At this stage, the introducer assembly 100 is fully assembled, and ready for introduction into the patient. The prosthesis 120 is retained at one end by the cylindrical sleeve 110 and the other by the distal attachment sections 140, and compressed by the sheath 130. If an aortic aneurism is to be repaired, the introducer assembly 100 can be inserted through a femoral artery over the guide wire, and positioned by radiographic techniques, which are not discussed here.

Once the introducer assembly 100 is in the desired deployment position, the sheath 130 is withdrawn to just proximal of the distal attachment section 140. This action releases the middle portion of the prosthesis 120 so that it can expand radially. Consequently, the prosthesis 120 can still be rotated or lengthened or shortened for accurate positioning. The proximal self-expanding stent 121, however, is still retained within the cylindrical sleeve 110. Also, the distal end 142 of the prosthesis 120 is still retained within the external sheath 130.

Next, the pin vise 139 is released to allow small movements of the thin walled tube 115 with respect to the thick walled tube 141. These movements allow the prosthesis 120 to be lengthened or shortened or rotated or compressed for accurate placement in the desired location within the lumen. X-ray opaque markers (not shown) may be placed along the prosthesis 120 to assist with placement of the prosthesis.

When the proximal end of the prosthesis 120 is in place, the proximal trigger wire is withdrawn by distal movement of the proximal wire release mechanism 124. The proximal wire release mechanism 124 and the proximal trigger wire can be completely removed by passing the proximal wire release mechanism 124 over the pin vise 139, the screw cap 146, and the connection means 116.

Next, the screw cap 146 of the pin vise 139 is then loosened. After this loosening, the thin walled tube 115 can be pushed in a proximal direction to move the cylindrical sleeve 110 in a proximal direction. When the cylindrical sleeve 110 no longer surrounds the self-expanding stent 121, the self-expanding stent 121 expands. When the self-expanding stent 121 expands, the barbs 151 grip the walls of the lumen to hold the proximal end of the prosthesis 120 in place. From this stage on, the proximal end of the prosthesis 120 cannot be moved again.

Once the proximal end of the prosthesis 120 is anchored, the external sheath 130 is withdrawn distally of the distal attachment section 140. This withdrawal allows the contralateral limb 160 and the longer leg 132 of the prosthesis 120 to expand. At this point, the distal end 142 of the prosthesis 120 may still be moved. Such positioning of the prosthesis 120 may ensure that the shorter leg 160 extends in the direction of a contralateral artery.

After the shorter leg 160 extends in the direction of the contra-iliac artery, the tubular prosthesis 150 is deployed. The tubular prosthesis 150 is deployed such that it forms a connection with the shorter leg 160 and extends from the shorter leg 160 into the contralateral artery.

There are many ways of practicing the invention. For instance, while each bottom apex in the anchoring stent has been depicted with multiple sutures using three or more openings or penetrations, it is not necessary to so reinforce all the apices. If it is desired to add stitches or sutures in only one apex or a portion of the periphery of the stent or graft, that may also be done. In addition, the openings or penetrations may be prepared in advance in the graft, such as by punching or other preparatory method for forming openings or penetrations.

Some anchoring stents are referred to as "top" stents, but an anchoring or top or "bare" stent need not be vertically on top of the graft or another stent; an anchoring stent may be vertically higher or lower than a neighboring stent. Depending on the application, an anchoring stent may be placed horizontally side by side another stent in a stent-graft or other endoluminal prosthetic device. All such devices and methods may be equivalent to the inventions described above and claimed in the claims attached hereto. The embodiments depicted herein include anchoring stents, but embodiments of the invention in which two or more stitches share a hole or a penetration in a graft are not limited to anchoring stents. Embodiments may include an internal or an external stent, alone or with another stent.

Any other undisclosed or incidental details of the construction or composition of the various elements of the disclosed embodiment of the present invention are not believed to be critical to the achievement of the advantages of the present invention, so long as the elements possess the attributes needed for them to perform as disclosed. The selection of these and other details of construction are believed to be well within the ability of even one of rudimentary skills in this area, in view of the present disclosure.

Illustrative embodiments of the present invention have been described in considerable detail for the purpose of disclosing a practical, operative structure whereby the invention may be practiced advantageously. The designs described herein are intended to be exemplary only. The novel characteristics of the invention may be incorporated in other structural forms. The invention encompasses embodiments both comprising and consisting of the elements described herein with reference to the illustrative embodiments. Unless otherwise indicates, all ordinary words and terms used herein shall take their customary meaning as defined in the New Shorter Oxford English Dictionary, 1993 edition. All technical terms not defined herein shall take on their customary meaning as established by the appropriate technical discipline utilized by those normally skilled in that particular art area. All medical terms shall take their meaning as defined by Stedman's Medical Dictionary, 27th edition.

## Claims

1. An endoluminal prosthetic device (10), comprising:
a graft (12) comprising warp and weft threads, and
a stent (14) comprising a plurality of struts (15) and apices (16) between the struts, **characterised in that** at least one of the apices is secured to the graft by at least two stitches (18) between the threads of the graft and within five graft threads of each other.

2. The endoluminal prosthetic device of Claim 1, wherein at least two of the stitches (35b, 35c, 36) form a running suture (37, 38).

3. The endoluminal prosthetic device of Claim 1, wherein the stent (14) is a zigzag stent.

4. The endoluminal prosthetic device of Claim 1, wherein the graft (12) is made of material selected from the group consisting of polyester, polyurethane, polyethylene, polypropylene and polyetrafluoroethylene.

5. The endoluminal prosthetic device of Claim 1, wherein the stitches (18) further comprise monofilament or braided multi-filament material selected from the group consisting of polyester, nylon, polyaramid, polypropylene, and polyethylene.

6. The endoluminal prosthetic device of Claim 1, whereby the stent (14) comprises material selected from the group consisting of stainless steel, titanium, and shape memory material.

7. The endoluminal prosthetic device of Claim 1, further comprising a cuff (13) on the graft, and wherein the penetrations (19) is through both layers of the cuff.

8. The endoluminal prosthetic device of Claim 1, wherein the graft (12) incorporates biomaterial selected from the group consisting of collagen, extracellular matrix material, and small intestine submucosal (SIS).

9. The endoluminal prosthetic device of Claim 2, further comprising a second stent (21), at least one of the apices in the second stent secured to the graft by stitches in the running suture.

10. The endoluminal prosthetic device of claim 1, wherein the stent (14) is an anchoring stent and the device further comprises a second stent (21) secured with stitches (28) to the graft.

11. The endoluminal prosthetic device of Claim 10, wherein the device is an aortic graft and the anchoring stent (14) is a suprarenal stent.

12. A method of forming an endoluminal prosthetic device (10), the method comprising:
providing a graft (12) having warp and weft threads; and
joining a first stent (14) to the graft, the first stent comprising a plurality of struts (15) and apices (16) between the struts, **characterised in that** the first stent is joined to the graft by at least two stitches (18) between the threads of the graft and within five graft threads of each other.

13. The method of Claim 12, further comprising linking at least two stitches with a running suture.

14. The method of Claim 12, further comprising joining a second stent (21) to the graft, at least one of the apices in the second stent being secured to the graft by stitches in a suture (37, 38) running to the first stent.

15. The method of Claim 12, further comprising folding the graft to form a cuff (13) before the step of joining the first stent.

16. The method of Claim 15, further comprising forming at least one opening (19) in the graft before the step of joining.

## Patentansprüche

1. Endoluminale Prothesenvorrichtung (10), die Folgendes umfasst: eine Graftprothese (12) mit Kett- und Schussfäden, und einen Stent (14) mit einer Vielzahl von Streben (15) und Spitzen (16) zwischen den Streben, **dadurch gekennzeichnet, dass** zumindest eine der Spitzen mit zumindest zwei Stichen (18) innerhalb von fünf Graftprothesenfäden voneinander an der Graftprothese befestigt ist.

2. Endoluminale Prothesenvorrichtung nach Anspruch 1, worin zumindest zwei der Stiche (35b, 35c, 36) eine fortlaufende Naht (37, 38) bilden.

3. Endoluminale Prothesenvorrichtung nach Anspruch 1, worin der Stent (14) ein Zickzack-Stent ist.

4. Endoluminale Prothesenvorrichtung nach Anspruch 1, worin die Graftprothese (12) aus einem Material besteht, das ausgewählt ist aus der Gruppe von Polyester, Polyurethan, Polyethylen, Polypropylen und Polytetrafluorethylen.

5. Endoluminale Prothesenvorrichtung nach Anspruch 1, worin die Stiche (18) ferner ein monofiles oder geflochtenes multifiles Material umfassen, das ausgewählt ist aus der Gruppe von Polyester, Nylon, Polyaramid, Polypropylen und Polyethylen.

6. Endoluminale Prothesenvorrichtung nach Anspruch 1, worin der Stent (14) ein Material umfasst, das ausgewählt ist aus der Gruppe von Edelstahl, Titan und Formgedächtnismaterial.

7. Endoluminale Prothesenvorrichtung nach Anspruch 1, die ferner ein Cuff (13) auf der Graftprothese umfasst und worin die Penetrationen (19) durch beide Cuffschichten gehen.

8. Endoluminale Prothesenvorrichtung nach Anspruch 1, worin die Graftprothese (12) ein biologisches Material enthält, das ausgewählt ist aus der Gruppe von Kollagen, extrazellulärem Matrixmaterial und Dünndarm-Submukosa (SIS).

9. Endoluminale Prothesenvorrichtung nach Anspruch 2, das ferner einen zweiten Stent (21) umfasst, wobei zumindest eine der Spitzen im zweiten Stent durch Stiche in der fortlaufenden Naht an der Graftprothese befestigt ist.

10. Endoluminale Prothesenvorrichtung nach Anspruch 1, worin der Stent (14) ein Verankerungsstent ist und die Vorrichtung ferner einen zweiten Stent (21) umfasst, der mit Stichen (28) an der Graftprothese befestigt ist.

11. Endoluminale Prothesenvorrichtung nach Anspruch 10, worin die Vorrichtung eine Aortengraftprothese und der Verankerungsstent (14) ein suprarenaler Stent sind.

12. Verfahren zur Bildung einer endoluminalen Prothesenvorrichtung (10), wobei das Verfahren Folgendes umfasst: Bereitstellung einer Graftprothese (12) mit Kett- und Schussfäden; und Verbindung eines ersten Stents (14) mit der Graftprothese, wobei der erste Stent eine Vielzahl von Streben (15) und Spitzen (16) zwischen den Streben umfasst, **dadurch gekennzeichnet, dass** der erste Stent mit zumindest zwei Stichen (18) innerhalb von fünf Graftprothesenfäden voneinander mit der Graftprothese verbunden ist.

13. Verfahren nach Anspruch 12, das ferner die Verbindung von zumindest zwei Stichen mit einer fortlaufenden Naht umfasst.

14. Verfahren nach Anspruch 12, das ferner die Verbindung eines zweiten Stents (21) mit der Graftprothese umfasst, wobei zumindest eine der Spitzen im zweiten Stent mit Stichen in einer Naht (37, 38), die zum ersten Stent verläuft, an der Graftprothese befestigt ist.

15. Verfahren nach Anspruch 12, das ferner das Falten der Graftprothese zur Bildung eines Cuffs (13) vor dem Schritt der Verbindung des ersten Stents umfasst.

16. Verfahren nach Anspruch 15, das ferner die Bildung zumindest einer Öffnung (19) in der Graftprothese vor dem Verbindungsschritt umfasst.

## Revendications

1. Dispositif (10) de prothèse endoluminale comprenant :
un greffon (12) qui contient des fils de chaîne et des fils de trame et
un tuteur (14) qui comprend plusieurs entretoises (15) et plusieurs sommets (16) entre les entretoises, **caractérisé en ce que**
au moins l'un des sommets est fixé au greffon par au moins deux piqûres (18) à distance mutuelle de cinq fils du greffon.

2. Dispositif de prothèse endoluminale selon la revendication 1, dans lequel au moins deux des piqûres (35b, 35c, 36) forment une suture continue (37, 38).

3. Dispositif de prothèse endoluminale selon la revendication 1, dans lequel le tuteur (14) est un tuteur en zigzag.

4. Dispositif de prothèse endoluminale selon la revendication 1, dans lequel le greffon (12) est réalisé en un matériau sélectionné dans l'ensemble constitué du polyester, du polyuréthane, du polyéthylène, du polypropylène et du polytétrafluoroéthylène.

5. Dispositif de prothèse endoluminale selon la revendication 1, dans lequel les piqûres (18) comprennent en outre un matériau en un filament ou en plusieurs filaments tressés sélectionné dans l'ensemble constitué du polyester, du nylon, du polyaramide, du polypropylène et du polyéthylène.

6. Dispositif de prothèse endoluminale selon la revendication 1, dans lequel le tuteur (14) comprend un matériau sélectionné dans l'ensemble constitué de l'acier inoxydable, du titane et d'un matériau à mémoire de forme.

7. Dispositif de prothèse endoluminale selon la revendication 1, comprenant en outre une coiffe (13) placée sur le greffon, les perforations (19) traversant les deux couches de la coiffe.

8. Dispositif de prothèse endoluminale selon la revendication 1, dans lequel le greffon (12) comprend un biomatériau sélectionné dans l'ensemble constitué du collagène, d'un matériau extracellulaire de matrice et de sous-muqueuse du petit intestin (SIS).

9. Dispositif de prothèse endoluminale selon la revendication 2, comprenant en outre un deuxième tuteur (21), au moins l'un des sommets du deuxième tuteur étant fixé au greffon par des piqûres formées dans la suture continue.

10. Dispositif de prothèse endoluminale selon la revendication 1, dans lequel le tuteur (14) est un tuteur d'ancrage et le dispositif comprend en outre un deuxième tuteur (21) fixé par des piqûres (28) au greffon.

11. Dispositif de prothèse endoluminale selon la revendication 10, dans lequel le dispositif est un greffon aortique et le tuteur d'ancrage (14) est un ancrage surrénal.

12. Procédé de formation d'un dispositif (10) de prothèse endoluminale, le procédé comprenant les étapes qui consistent à :
prévoir une greffe (12) qui présente des fils de chaîne et des fils de trame et
relier un premier tuteur (14) au greffon, le premier tuteur comprenant plusieurs entretoises (15) et plusieurs sommets (16) situés entre les entretoises, **caractérisé en ce que**
le premier tuteur est relié au greffon par au moins deux piqûres (18) formées à une distance mutuelle de cinq fils de greffon.

13. Procédé selon la revendication 12, comprenant en outre la liaison d'au moins deux piqûres avec une suture continue.

14. Procédé selon la revendication 12, comprenant en outre l'étape qui consiste à relier un deuxième tuteur (21) au greffon, au moins l'un des sommets du deuxième tuteur étant fixé au greffon par des piqûres dans une suture (37, 38) qui s'étend jusqu'au premier tuteur.

15. Procédé selon la revendication 12, comprenant en outre le pliage du greffon pour former une coiffe (13) avant l'étape de jonction de la première suture.

16. Procédé selon la revendication 15, comprenant en outre la formation d'au moins une ouverture (19) dans le greffon avant l'étape de jonction.
